(19) Europäisches Patentamt
European Patent Office
Office européen des brevets

(11) **EP 2 152 685 B1**

## (12) EUROPEAN PATENT SPECIFICATION

(45) Date of publication and mention
of the grant of the patent:
**25.01.2017 Bulletin 2017/04**

(21) Application number: **08743366.0**

(22) Date of filing: **28.04.2008**

(51) Int Cl.:
*A61Q 19/02* *(2006.01)*     *A61K 8/34* *(2006.01)*

(86) International application number:
**PCT/US2008/005459**

(87) International publication number:
**WO 2008/153629 (18.12.2008 Gazette 2008/51)**

(54) **SKIN LIGHTENING COMPOSITIONS AND METHODS**

HAUTAUFHELLENDE ZUSAMMENSETZUNGEN UND VERFAHREN

COMPOSITIONS ET PROCÉDÉS D'ÉCLAIRCISSEMENT DE LA PEAU

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **06.06.2007 US 810626**

(43) Date of publication of application:
**17.02.2010 Bulletin 2010/07**

(73) Proprietor: **Sytheon Ltd.
Lincoln Park, NJ 07035 (US)**

(72) Inventor: **CHAUDHURI, Ratan, K.
Lincoln Park, NJ 07035 (US)**

(74) Representative: **Tomkins & Co
5 Dartmouth Road
Dublin 6 (IE)**

(56) References cited:
EP-A1- 0 341 664     DE-C- 950 913
US-A- 4 959 393     US-A1- 2006 129 002
US-A1- 2007 098 655

• **Anonymous: "Synovea HR & Asyntra SL Safe &
Highly Effective in Controlling Skin
Pigmentation", , 11 March 2011 (2011-03-11),
XP055224187, Retrieved from the Internet:
URL:http://www.in-cosmetics.com/__novadocu
ments/70127?v=635566846023600000 [retrieved
on 2015-10-28]**
• **Anonymous: "Description of a Proposed
Reference Dose Resorcinol", , 1 September 2004
(2004-09-01), XP055300873, Retrieved from the
Internet:
URL:http://www.dep.state.pa.us/dep/subject
/advcoun/cleanup/2004/Dec8/AMEC_Resorcinol
RFD_090804.pdf [retrieved on 2016-09-08]**

Remarks:
The file contains technical information submitted after
the application was filed and not included in this
specification

**Description**

**FIELD OF THE INVENTION**

This invention relates to skin lightening compositions for lightening normal and/or hyper-pigmented skin comprising (i) highly purified hexylresorcinol, (ii) optionally, though preferably, at least one other skin lightening agent and (iii) a dermatologically acceptable carrier. Preferably, the said hexylresorcinol is free or substantially free from resorcinol and/or other non-intended phenols and has a purity of over 96% w/w, most preferably at least 99% w/w. These compositions are especially effective for skin lightening/even toning and can also be used in compositions for preventing or reducing formation of sun-, laser therapy-, acne- and scar-induced hyper-pigmented spots as well as age spots, liver spots, freckles, melasma etc.

**BACKGROUND OF THE INVENTION**

Human skin color is quite variable around the world. It ranges from a very dark brown among some Africans, Australians and Asian-Indians to a near pinkish yellow among some northwest Europeans. There are no people who truly have black, white, red or yellow skin. These are commonly used terminologies that do not reflect biological reality. Skin coloration in humans arises from a complex series of cellular processes that are carried out within that population of cells known as the melanocytes located in the lower part of the epidermis. These processes result in the synthesis and transfer of a pigment, melanin, which, besides being responsible for skin color and tone, is the key physiological defense against sun-induced damage, such as sunburn, photoaging and photocarcinogenesis.

The mechanism by which melanin is produced is known as melanogenesis. The so formed melanin is accumulated/deposited in melanosomes, vesicles found within the melanocyte cells, which are subsequently transferred from the melanocytes and taken up and internalized by the keratinocytes, which then carry them to the surface of the skin. Generally speaking, skin coloration is primarily regulated by the amount and type of melanin synthesized by the epidermal melanocyte. However, additional and equally contributing factors include (a) the efficiency of the transfer of the melanosomes, hence the melanin, from the melanocytes to the neighboring keratinocytes and (b) the subsequent distribution and degradation of the transferred melanosomes by the recipient keratinocytes. Environmental factors can also markedly affect skin color. For example, exposure of the skin to ultraviolet light markedly influences and increases the amount and rate of melanin production, most often producing a further darkening of the skin or a "tan." Conversely, exposure to other factors, especially agents that interfere with melanin production and/or the transfer of melanin, may result in a decrease of melanin production and/or the rate or efficiency of its transfer resulting in a lightening of the skin.

Hyperpigmentation, hypopigmentation, and other pigmentation disorders are quite common and can arise from a number of causes including diet, medications and the like. Common pigmentation disorders include melasma (dark patches experienced in pregnancy) and liver spots (which often develop with age), and may arise as a side effect of birth control pills, and/or as a persistent result of acne, burns, bites and other skin injuries, and vitiligo. Similarly, freckles, chloasma and pigmentary deposits after sun exposure tend to occur or increase or become difficult to disappear with increasing age, thus being one of the more disconcerting and/or common problems of skin care for persons of middle to advanced age. Post inflammatory hyper-pigmentation is also found to occur following after laser therapy.

In an effort to address such pigmentation disorders, various preparations have been formulated for use in the treatment of age spots and freckles or to obtain even-toning effects. Such treatments are not; however, limited for use in treating disorders but are also used in some cultures/markets merely for the purpose of changing or modifying ones natural skin color. Such treatments are typically referred to by a number of different terminologies including "skin lightener", "skin whitener", "skin even-toner" and "skin brightener". The specific terminology used is oftentimes a matter of regulatory controls; rather than one of performance or application. For example, "skin whitening" terminology is very commonly used in Asia whereas such terminology is not allowed under US Food and Drug Administration regulations. Other terminologies are commonly used as well including melanin inhibitory agents, depigmenting agents, tyrosinase inhibitors (tyrosinase being the key enzyme responsible for melanin synthesis), etc. Whatever terminology is employed, the general premise is that they all relate to a reduction in the formation or rate of formation of melanin. In this patent, the 'skin lightener' and 'even-toner' terminologies will be used as they are physiologically more relevant.

A number of agents and methods for skin lightening have been developed and put on the market. Such methods include the oral administration of large doses of Vitamin C, the parenteral administration of glutathione, the topical administration of peroxide bleaching agents such as hydrogen peroxide, zinc peroxide, sodium peroxide and the like, and the topical application of Vitamin C and/or cysteine. Vitamin C, however, has stability issues, especially in water based formulations, resulting in color and odor changes. Thiol compounds such as glutathione and cysteine have slow and/or generally poor depigmentation performance properties.

Perhaps the most commonly employed depigmentation agent has been hydroquinone and its derivatives. However, these compounds, while effective, have serious, detrimental side effects. Even at concentrations below 2%, hydroquinone is both irritating and cytotoxic to the melanocytes. With the growing concern as to their safety, hydroquinone and its derivatives are largely being phased out of use or banned altogether in topical applications. Similar problems have been experienced with Kojic acid depigmentation agents as well.

A wide-range of polyphenols present in plant extracts have also been used for skin lightening/even-toning purposes. Melanin inhibitory activity of natural polyphenols, such as, anthraquinones (K Jones, J Hughes, M Hong, Q Jia, S Orndorff, Modulation of melanogenesis by aloeosin: a competitive inhibitor of tyrosinase, Pigment Cell Research, 15, 335-340, 2002), arylbenzofurans (SH Lee, SY Choi, H Kim, JS Hwang, BG Lee, Mulberoside F isolated from the leaves from the leaves of Murus alba inhibits melanin biosynthesis, Bio Pham Bull, 25, 1045-1048, 2002), chalcones (O Nerya, R Musa, S Khatib, S Tamir, J Vaya, Chalcones as potent tyrosinase inhibitors: the effect of hydroxyl positions and numbers, Phytochem, 65, 1389-1395, 2004), coumarins (Y Masamoto, Y Murata, K Baba, Y Shimoishi, M Tada, K Takahata, Inhibitory effects of esculetin on melanin biosynthesis, Biol Pharm Bull, 27, 422-425, 2004), flavonoids (Y Yokoto, H Nishio, Y Kubota, M Mizoguchi, The inhibitory effect of glabridin from licorice extracts on melanogenesis and inflammation, Pigment Cell Research, 11, 355-361, 1998; JK No, DY Soung, YJ Kim, KH Shim, YS Jun, SH Rhee, R Yokozawa, HY Chung, Inhibition of tyrosinase by green tea components, Pharmacol Letters, 65, 241-246, 1999;O Nerya, J Vaya, R Musa, S Izrael, R Ben-Arie, S Tamir, Glabrene and Isoliquiritigenin as tyrosinase inhibitors fro Licorice roots, J Agr Food Chem, 51, 1201-1207, 2003; I Kubo, I Kinst-Hori, SK Chaudhuri, Y Kubo, Y Sanchez, T Ogura, Flavonols from Heterotheca inuloides: Tyrosinase inhibitory activity and structural criteria, Bioorganic & Medicinal Chemistry, 8, 1749-1755, 2000), Stilbenes (NH Shin, SY Ryu, EJ Choi, SH Kang, IM Chang, KR Min, R Kim, Oxyresveratrol as the potent inhibitor on dopa oxidase activity on mushroom tyrosinase, Biochem Biophys Res Commun, 243, 801-803, 1998; YM Kim, J Yun, CK Lee, H Lee, KR Min, Y Kim, Oxyresveratrol and hydroxystilbene compounds, J Biol Chem, 277, 16340-16344, 2002); low molecular tannins (RK Chaudhuri, Z Lascu and G Puccetti, Inhibitory effects of Phyllanthus emblica tannins on melanin synthesis, Cosmetics & Toiletries, 122(2), 73-80, 2007) have been reported. Exemplary patents that describe the use of natural and synthetic phenolic compounds as skin lighteners include: US 6,649,150 - Chaudhuri et al.; US 6,969,509 - Chaudhuri et al.; US 5,670,154 - Hara et al.; and US 5,880314 - Shinomiya et. al.

One class of polyphenolic compounds that has received a lot of attention, at least in the patent literature, is that based on substituted resorcinols and their derivatives. Early applications, including US 4,959,393 - Torihara et. al., employed n-alkyl substituted resorcinol, especially those based on $C_2$ to $C_{12}$ n-alkyl substituted resorcinol. Subsequent applications, including JP 5-04905 - Hamazaki et. al. and WO 2006/049184 - Fukunishi et. al., focused on compositions containing 4-alkylresorcinol derivatives including, straight chain and branched, $C_2$ to $C_{12}$ n-alkyl substituted resorcinols and their salts. Others still employed such 4-alkylresorcinols, especially n-butylresorcinol, in combination with certain branched polymers, e.g., acrylic acid-alkyl methacrylate, JP 2001-010925 - Seto et. al.

Though early activity seemed to focus on the simple alkyl substituted resorcinols, much greater focus has more recently been directed to more complex hydrocarbyl and/or hetero moiety substituted resorcinols. Hetero-substituted resorcinols include the thio, thiane (especially dithiane), amide, amine, keto and carboxylic substituted resorcinols as shown in US 5,468,472 - LaGrange et. al.; US 6,875,425 - Harichian et. al., US 6,852,310 - Harichian et. al.; and JP 1125563 - Sakai. Perhaps the greatest attention has focused on the more complex hydrocarbyl substituted resorcinols, specifically, the cycloalkyl resorcinols and substituted derivatives thereof. Such skin lightening agents are more fully described in, e.g., US 2006/0257340 - Nair; US 6,878,381 - Collington; US 6,933,319 - Browning et. al.; US 6,852,747 - Bradley et. al.; US 6,828,460 - Browning et. al.; US 6,797,731 - Bradley et. al.; US 6,590,105 - Bradley et. al.; US 6,541,473 - Bradley et. al.; and US 6,132,740 - Hu.

Despite the significant focus on substituted resorcinols and their derivatives, they too are not without their problems. For example, despite their relatively good skin lightening capabilities, they tend to suffer from stability issues, particularly color stability, rendering then generally unsuitable for topical applications. While the stability issues are most severe with the straight chain and branched alkyl substituted resorcinols, they are not limited thereto. Indeed, many, if not most, phenolic based skin lightening agents, whether synthetic or natural extracts, are susceptible to air and/or UV oxidation: thus leading to color instability which also oftentimes coincides with loss of skin lightening efficacy. In following, efforts have been undertaken to improve their stability by the incorporation of various additives including metal oxides (US 6,863,897 - Love et. al.) and terpenoids (US 6,858,217 - Kerschner et. al.); however, their success has been limited. Another significant detriment to the use of substituted resorcinols and their derivatives has been their relatively high level of byproducts and contaminants. Specifically, commercial grade resorcinols tend to be rather crude, containing significant levels of other polyphenols as well as resorcinol itself, owing to their relatively inefficient production processes and syntheses. For example, commercial grade $C_2$ - $C_{12}$ alkyl-resorcinols are typically only on the order of 64-86% purity. The high level of impurities only adds to the stability concerns. More importantly, the presence of resorcinol and other undesired phenols and polyphenols also add concerns of skin irritancy and sensitization problems as well as other skin and health concerns. For example, resorcinol is a known skin irritant and sensitizer and has been associated with producing allergic dermatitis in a small proportion of individuals exposed repeatedly to resorcinol-containing cosmetic and pharmaceutical products. Resorcinol has also been found to be irritating to the eyes, the skin and the respiratory tract and is suspected of causing effects on the blood, resulting in formation of methaemoglobin. Although some of the more complex resorcinol derivatives mentioned above, especially the cycloalkyl substituted resorcinols, may have higher purity and, thus, avoid or lessen these concerns, they are oftentimes found to be less effective as skin lightening agents.

There remains a need for skin lightening agents that do not suffer from instability, especially oxidative instability that affects the color and efficacy of the skin lightening composition and the cosmetic/treatment formulation into which the skin lightening agents are incorporated.

There remains a need for skin lightening agents that do not possess or raise concerns relative to skin irritancy and sensitization, or other possible skin or health consequences.

In general, there remains a need for additional skin lightening agents, especially ones of high efficacy. Most especially, there remains a need for skin lightening agents that are highly efficacious, stable and non-irritating.

Further, there is also a need for skin lightening agents that are compatible with and, most preferably, work synergistically with other skin lightening agents, especially in ways that enable the use of less and less skin lightening actives without compromising efficacy.

Finally, there remains a need for skin lightening compositions that achieve any or all of the foregoing objectives formulations and that are easy to use with highly efficacious results. In particular there remains a need for skin lightening compositions based on synergistic combinations of skin lightening agents or actives wherein the overall amount of the agents to be used are less than would be needed with either agent on their own.

## SUMMARY

According to the present invention there are provided novel skin lightening compositions comprising (i) highly purified hexylresorcinol, (ii) optionally, though preferably, at least one other skin lightening agent, and (iii) a dermatologically acceptable carrier. The hexylresorcinol has a purity of at least 96% w/w, most preferably over 99% w/w, with less than 0.1% w/w resorcinol, preferably below 0.05%. These compositions are especially effective for skin lightening/even toning, especially as compared to similar compositions made with conventional, commercial grade substituted hexylresorcinol, i.e., that of less than 86% purity.

While the aforementioned formulations are effective as skin lightening agents, they are also suitably employed as preventative compositions to be applied routinely, especially daily, for preventing the formation of sun-induced or laser therapy-induced or scar-induced hyper-pigmented spots as well as that resulting from other factors including diet and/or pharmaceutical agents.

The skin lightening compositions of the present invention will typically comprise highly purified hexylresorcinol in an amount of from about 0.01 to about 20 wt %, preferably from about 0.05 to about 10 wt %, most preferably from about 0.1 to about 5% wt % based on the total weight of the formulation. When used in combination with other conventional skin lightening agents, the second skin lightening agent will be present in ranges typical for that agent, generally on the order of from about 0.01 to about 20 wt %, preferably from about 0.05 to about 10 wt %, most preferably from about 0.1 to about 5 wt %. Where synergy is found, though the aforementioned ranges still apply, even less of the second skin lightening agent may be used, most preferably in the range of from about 0.1 to about 2.5 wt %. Similarly, the weight ratio of the two skin lightening agents will vary depending upon the second skin lightening agent; however, they will generally be present in a weight ratio of 20:1 to 1:20, preferably from 10:1 to 1:10, more preferably 5:1 to 1:5, most preferably 2:1 to 1:2. These skin lightening agents are incorporated into conventional dermatologically acceptable carriers. Additionally, these skin lightening compositions may optionally include an effective amount of at least one skin protective and/or treatment ingredients such as sunscreens, antioxidants, vitamins, anti-inflammatory agents, moisturizers, emollients, humectants, and the like, and mixtures thereof, in their conventional amounts.

The skin lightening compositions of the present invention are applied topically and may take the form of a cream, lotion, spray, ointment, gel, or other any other topically applicable form.

## DETAILED DESCRIPTION OF THE INVENTION

All patents, patent publications, and technical articles referenced herein are hereby incorporated herein in their entirety.

As used herein and in the appended claims, the phrase "substantially free of" means that the recited compound or component, if present, is present at an inconsequential level, generally less than 0.1 wt% based on the weight of hexylresorcinol. Most preferably, the amount, if present will be insufficient to manifest any skin irritation or sensitization following topical application: in such regard, it will be as if the same formulation were completely free of the recited compound.

As used herein the term "dermatologically-acceptable" means that the compositions or components thereof so described are suitable for use in contact with human skin without undue toxicity, incompatibility, instability, irritability, allergic response, and the like.

As used herein the term "topical" or "topically" refers to the application of the composition of the present invention onto the surface of the skin or a portion thereof.

As used herein the term "safe and effective amount" means an amount of a compound or composition sufficient to significantly induce a positive benefit, preferably a positive keratinous tissue appearance or feel benefit, including independently or in combination the benefits disclosed herein, but low enough to avoid serious side effects.

As used herein the term "post-inflammatory hyperpigmentation" refers to the changes in melanin content as a response to an inflammatory event (e.g., acne, scratch, laser therapy, insect sting or bite, sunburn, etc), especially in individuals of darker skin tone or color.

The principal and critical element of the skin lightening compositions of the present invention is purified hexylresorcinol. Purified hexylresorcinol is characterized as being at least 96% pure, w/w, preferably at least 99% pure, w/w, and is preferably substantially free of resorcinol, most preferably substantially free of resorcinol and other phenols that are suspected of being irritants or sensitizers. Specifically, the hexylresorcinol will have less than 0.1 wt %, most preferably less than 0.05 wt %, resorcinol. Purified alkyl resorcinols and methods of their production are described in, for example, US 2006/0129002 A1 - Wassmann-Wilken et. al. Highly purified hexylresorcinol suitable for use in the practice of the present invention may be prepared as follows: Resorcinol and hexanoic acid are reacted in the presence of zinc chloride catalyst to produce hexanoylresorcinol. To drive the equilibrium towards the formation of hexanoylresorcinol, the water formed during the reaction was continuously removed by azeotropic distillation using solvents like xylene, toluene, etc., which leads to almost total conversion of resorcinol to hexanoylresorcinol. The crude product was subjected to vacuum distillation which resulted in a product whose resorcinol content was found to be < 0.01% to nil. The resultant hexanoylresorcinol is then dissolved in ethanol and subjected to Clemenson reduction to obtain the 4-Hexylresorcinol which is then crystallized from hexane to give a product having a resorcinol content of <0.005% to nil. The highly purified hexylresorcinol employed in the examples of this application is available from Sytheon Ltd. of Lincoln Park, NJ 07035 under the tradename Synovea™ HR. This product generally conforms to the product specification set forth in Table 1.

| Table 1 - Synovea™ HR hexylresorcinol | |
|---|---|
| Analytical Profile | Specifications |
| Assay for hexylresorcinol (HPLC) | $\geq$ 99% (w/w) |
| Identity (IR) | To match with standard |
| Melting range | 62 to 67 °C |
| Moisture content | $\leq$1% |
| Sulfated ash | $\leq$0.1% |
| Resorcinol | $\leq$0.1% |
| Heavy Metals | |
| Lead (Pb) | <5 ppm |
| Arsenic (As) | <2 ppm |
| Mercury (Hg) | <1 ppm |
| Microbiological profile | |
| Total aerobic plate count | $\leq$100 CFU/g |
| Yeast and mold count | $\leq$10 CFU/g |
| *Escherichia coli* | Absent in 1 g |
| *Salmonella* | Absent in 10 g |

The highly pure hexylresorcinol will be present in the skin lightening in a safe and effective amount, generally from about 0.01 wt % to about 20 wt %, preferably from about 0.05 to about 10 wt %, more preferably from about 0.1 to about 5% wt %, most preferably from about 0.1 to about 2.5 wt %, based on the total weight of the formulation.

Optionally, though preferably, the skin lightening compositions of the present invention will also contain a second skin lightening/even-toning ingredient other than hexylresorcinol. Although it may not be possible to list all known skin lightening agents, the following non-limiting suitable examples may be mentioned - coconut water, coconut milk, palm water, palm nut milk, pecan nut milk, almond nut milk, cashew nut milk, walnut nut milk, and concentrates of the foregoing, or any combinations of the foregoing. Other non-limiting skin lightening ingredients are, for example, *Phyllanthus emblica* fruit extract, bearberry extract, Mulberry extract, Licorice extract, Propolis extract, aceroal cherry fermentate, cucumber extract, Green tea poly phenols, Grape seed extract, Pine bark polyphenols, resveratrol, oxyresveratrol, stilbenes, coumarins, flavonoids, niacinamide, anthraquinones, xanthones, lignans, glabridin, curcumine, dihydrocucurmine, ep-igallocatechin-3-gallate, hydroxyl benzoic acids or their derivatives, tomato glycolipids, perilla plant, ligusticum lucidum extract, and combinations of any two or of the foregoing.

Suitable skin lightening agents also include the sugar amines, which are also known as amino sugars and are to be employed in a safe and effective amount. The sugar amine compounds useful in the present invention are described U.S. Pat. No. 6,159,485. Sugar amines can be synthetic or natural in origin and can be used as pure compounds or mixtures of compounds (e.g., extracts from natural sources or mixtures of synthetic materials). Glucosamine is generally found in many shellfish and can also be derived from fungal sources. As used herein, "sugar amine" includes isomers and tautomers of such and its salts (e.g., HCl salt) and is commercially available from Sigma Chemical Co. Examples of sugar amines that are useful herein include glucosamine, N-acetyl glucosamine, glucosamine sulfate, mannosamine, N-acetyl mannosamine, galactosamine, N-acetyl galactosamine, their isomers (e.g., stereoisomers), and their salts (e.g., HCl salt). Preferred ingredients are glucosamine, particularly D-glucosamine and N-acetyl glucosamine, particularly N-acetyl-D-glucosamine. Yet another group of skin lightening agents are the N-acyl amino acid compounds, including, but are not limited to, N-acyl phenylalanine, N-acyl tyrosine, their isomers, including their D and L isomers, salts, derivatives, and mixtures thereof. An example of a suitable N-acyl amino acid is N-undecylenoyl-L-phenylalanine is commercially available under the tradename Sepiwhite™ from Seppic (France). The skin lightening agents of this paragraph may be used alone or in combination with other secondary skin lightening agents mentioned above.

Like the hexylresorcinol, the second skin lightening agent will be present in a safe and effective amount, generally an amount sufficient to induce the desired effect of lightening. The specific amount will vary depending upon the type of agent and the nature and level of desired effect. However, the lightening agents are typically be present in an amount of from about 0.01 wt % to about 20 wt %, preferably from about 0.05 wt % to about 10 wt %, more preferably about 0.1 wt % to about 5 wt %, and most preferably about 0.1 wt % to about 2.5 wt %, based on the total weight of the composition. Similarly, the weight ratio of the two skin lightening agents will vary depending upon the nature of the second skin lightening agent and the specific result desired. Generally, however, the weight ratio of the highly purified hexylresorcinol to the second skin lightening agent or combination of agents will be from 20:1 to 1:20, preferably from 10:1 to 1:10, more preferably 5:1 to 1:5, most preferably 2:1 to 1:2.

Surprisingly, we have found that highly pure hexylresorcinol of the present invention significantly reduced or eliminated the discoloration, especially the browning effect, oftentimes associated with skin lightening agents, especially those based on or containing phenolic groups/moieties, such as, *Phyllanthus emblica* fruit extract (Emblica® of EMD Chemicals), Licorice, resveratrol etc. In addition, the combination of the purified hexylresorcinol with such secondary skin lightening agents oftentimes provides improved skin lightening properties as compared to either alone, even at the same total loading: thus enabling the use of less overall skin lightening agents for the same benefit. Given the concerns of skin irritancy and sensitivity, especially with prolonged, repetitive use of a product, any opportunity to reduce the amount of skin active agents is desirable and beneficial.

The third and final key component of the skin lightening/even-toning compositions of the present invention is the carrier. The carrier is that material or combination of materials that is used to essentially carry or deliver the skin lightening ingredient to the skin. The specific carrier material will depend upon the delivery method itself. For example, as mentioned earlier, the skin lightening/even-toning compositions may be in the form of lotions, creams, gels, foams, emulsions, dispersions, sprays, liposomes, coacervates, etc. Each composition will typically include any of the known topical excipients and like agents necessary for achieving the particular form; though it will be recognized that the components of such carriers will be or should be dermatologically-acceptable materials. Suitable excipients include, e.g., mineral oils and emulsifying agents. In its most simplest of embodiments, the carrier may be water, alcohol or water/alcohol combinations, or other solvent(s) or solvent systems in which the aforementioned actives may be, e.g., soluble, dispersed, emulsified, etc. Preferably, though, the skin lightening compositions will include excipients and the like that create a substantially stable, homogenous skin lightening/even-toning compositions and/or provide body and viscosity to the skin lightening/even-toning composition so that the actives do not merely run off the skin once applied. Typically, the carrier will comprise from about 30 to about 99 % by weight of the skin lightening composition.

Generally speaking, any known carrier or base composition employed in traditional cosmetic and/or dermatological applications/compositions may be used may be used in the practice of the present invention. Suitable carriers and carrier compositions are described at length in, for example, Gonzalez et. al. - US 7,186,404; Aust et. al. - US 7,175,834; Roseaver et. al.- US 7,172,754; Simoulidis et. al. - US 7,175,835; Mongiat et. al. - US 7,101,536; Maniscalco - US 7,078,022; Forestier et. al. US 5,175,340, US 5,567,418, US 5,538,716, and US 5,951,968; Deflandre et. al. - US 5,670,140; Chaudhuri - US 7,150,876, US 6,831,191, US 6,602,515, US 7,166,273, US 6,936,735, US 6,831,191, and US 6,699,463; Chaudhuri et. al. - US 6,165,450 and US 7,150,876; Bonda et. al. US 6,962,692; and Wang et. al. US 5,830,441 · Those skilled in the art will readily recognize and appreciate what carriers may be employed in light of the intended form and/or delivery method for the inventive skin lightening/even-toning compositions.

Though a carrier by itself is sufficient, the inventive skin lightening/even-toning compositions of the present invention may, and preferably will, contain various other components typically associated with skin care products. For example, various skin care agents including, but not limited to, conventional skin care excipients as well as additional photoprotective agents may be present. Such agents include, but are not limited to sunscreens, antioxidants, vitamins, anti-inflammatory agents, moisturizers, emollients, humectants, and the like, and mixtures thereof, in their conventional amounts. Exemplary agents and additive materials are described briefly below as well as in the aforementioned patents, especially Maniscalco - US 7,078,022.

Suitable antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, tocopherols (e.g., tocopherol acetate), tocotrienols, curcumin and its derivatives and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, *Phyllanthus emblica* and propolis. Other examples of antioxidants may be found on pages 1612 13 of the ICI Handbook as well as in Ghosal - US 6,124,268.

The skin lightening/even toning compositions of the present invention may also include one or more vitamins and/or their derivatives. Vitamins and vitamin derivatives include, for example, vitamin A, vitamin A propionate, vitamin A palmitate, vitamin A acetate, retinol, vitamin B, thiamine chloride hydrochloride (vitamin B.sub.1), riboflavin (vitamin B.sub.2), nicotinamide, vitamin C and derivatives (for example ascorbyl palmitate, ascorbyl glucoside, and ascorbyl acetate), vitamin D, ergocalciferol (vitamin D.sub.2), vitamin E, DL-$\alpha$-tocopherol, tocopherol E acetate, tocopherol hydrogensuccinate, vitamin K.sub.1, esculin (vitamin P active ingredient), thiamine (vitamin $B_1$), nicotinic acid (niacin), niacinamide, pyridoxine, pyridoxal, pyridoxamine, (vitamin $B_6$), pantothenic acid, biotin, folic acid and cobalamine (vitamin $B_{12}$). Preferred vitamins are, for example, vitamin A palmitate, vitamin C and derivatives thereof, DL-$\alpha$-tocopherol, tocopherol acetate, nicotinic acid, pantothenic acid and biotin. Vitamin E, which is often added to cosmetic and personal care products is also preferably stabilized by a suitable stabilizer according to the invention.

The skin lightening/even toning compositions of the present invention may also include suitable non-vitamin antioxidants, but are not limited to, BHT (butylated hydroxy toluene), L-ergothioneine (available as Thiotane™); tetrahydrocurcumin, cetyl pyridinium chloride, carnosine, diethylhexyl syrinylidene malonate (available as Oxynex® ST or Oxynex® ST Liquid available from EMD Chemicals/Merck, Germany.), ubiquinone (coenzyme Q10), Idebenone and combinations thereof.

Suitable emollients include those agents known for softening the skin which may be selected from hydrocarbons, fatty acids, fatty alcohols and esters. Petrolatum is a common hydrocarbon type of emollient conditioning agent. Other hydrocarbons that may be employed include alkyl benzoate, mineral oil, polyolefins such as polydecene, and paraffins, such as isohexadecane. Fatty acids and alcohols typically have from about 10 to 30 carbon atoms. Illustrative are myristic, isostearic, hydroxystearic, oleic, linoleic, ricinoleic, behenic and eruicic acids and alcohols. Oily ester emollients may be those selected from one or more of the following, triglyceride esters, acetoglyceride esters, ethoxylated glycerides, alkyl esters of fatty acids, ether esters, polyhydric alcohol esters and wax esters. Additional emollients or hydrophobic agents include $C_{12}$ to $C_{15}$ alkyl benzoate, dioctyladipate, octyl stearate, octyldodecanol, hexyl laurate, octyldodecyl neopentanoate, cyclomethicone, dicapryl ether, dimethicone, phenyl trimethicone, isopropyl myristate, caprylic/capric triglycerides, propylene glycol dicaprylate/dicaprate and decyl oleate, cyclomethicones and other silicone derivatives.

Suitable humectants include various polyhydric alcohols, especially polyalkylene glycols and, more preferably, alkylene polyols and their derivatives. Exemplary humectants include propylene glycol, dipropylene glycol, polypropylene glycol, polyethylene glycol, sorbitol, 2-pyrrolidone-5-carboxylate, hydroxypropyl sorbitol, hexylene glycol, ethoxydiglycol 1,3-butylene glycol, 1,2,6-hexanetriol, glycerin, ethoxylated glycerin, propoxylated glycerin, compatible solutes, such as ectoin, hydroxyectoin, taurines, carnitine, acetyl carnitine and mixtures thereof. When employed in effective amounts, generally from 1 to 30%, preferably from 2 to 20%, by weight of the skin lightening/even toning composition, these additives serve as skin moisturizers as well as reduce scaling and stimulate the removal of built-up scale from the skin.

The skin lightening/even-toning compositions of the present invention may also include one or more anti-inflammatory agent. Examples of anti-inflammatory ingredients include, but are not limited to, bisabolol, curcumin and its derivatives, retinoids, flavonoids, terpenes and other polyphenolics etc. These and other anti-inflammatory agents are disclosed in Gupta et. al. - US 2005/0048008A1.

Compositions containing steroidal anti-inflammatory, non-steroidal anti-inflammatory, as well as "natural" anti-inflammatory, such as extract of the plant Aloe vera, are also included in the present invention and have been disclosed for such use. See e.g., U.S. Pat. No. 4,185,100, Rovee, issued Jan. 22, 1980 (hydrocortisone, dexamethasone, naproxen, ketoprofen, ibuprofen); U.S. Pat. No. 4,338,293, Holick, issued Jul. 6, 1982 (steroidal anti-inflammatories); Law, et al., Br. J. Pharmac., 59(4), 591-597 (1977) (ibuprofen); Kaidbey, J. Invest. Dermatoloy, 66, 153-156 (1976) (indomethacin); and Gruber, et al., Clinical Pharm. and Therapeut., 13(1), 109-113 (1971) (aspirin, fenoprofen).

Additionally, the skin lightening/even toning compositions of the present invention may further contain and preferably do contain one or more sunscreen actives. Sunscreen actives are of two types, inorganic actives that work by reflecting the UV light and organic actives that work, predominately, by absorbing UV energy. The amount of the sunscreen active to be incorporated into the sunscreen formulations is that which is conventional in the art. Typically, the amount is dependent upon, among other factors, the delivery means, e.g., is it applied as a spray or lotion; the stability of the active; the efficacy of the selected sunblock active itself; and the application rate, as well as the particular SPF desired. From the commercial perspective, another factor influencing the level of such sunscreen actives in the sunscreen formulations is the regulatory limitations on their use. In the United States, for example, strict controls are placed upon the maximum level at which approved sunscreen actives may be present. Regulatory controls may also dictate which sunscreen actives may be used in which countries.

Suitable organic sunscreen actives include, for example, butyl methoxydibenzoylmethane (avobenzone), benzophenone-8, dioxybenzone, homosalate, octylsalate, menthyl anthranilate, octocrylene, ethyhexyl methoxycinnamate (Octinoxate), oxybenzone, ethylhexyl salicylate (Octisalate), benzophenone-3, ethylhexyl dimethyl PABA (Padimate O), glyceryl PABA, phenylbenzimidazole sulfonic acid, sulfisobezone, trolamine salicylate, 4-methylbenzylidene camphor, bisoctriazole, bemotrizinol, ecamsule, drometrizole trisiloxane, disodium phenyl dibenzimidazole tetrasulfonate, diethylamine hydroxybenzoyl hexyl bezoate, octyl triazone, hexyl benzoate, benzophenone-4, ethyhexyl triazone, diethylhexyl butamido triazone, bisimidazylate, polysilicone-15, etc.

Inorganic sunscreens include, but are not limited to, microfine surface treated titanium dioxide and microfine untreated and surface treated zinc oxide. The titanium dioxide in the sunscreen compositions preferably has a mean primary particle size of between 5 and 150 nm, preferably between 10 and 100 nm. Titanium oxide may have an anatase, rutile, or amorphous structure. The zinc oxide in the sunscreen compositions preferably has a mean primary particle size of between 5 nm and 150 nm, preferably between 10 nm and 100 nm.

Examples of suitable hydrophobically modified titanium dioxide compositions include but are not limited to: UV Titans® X161, M160, M262 (surface treated with stearic acid and alumina) (Kemira); Eusolex ® T-2000 (surface treated with alumina and simethicone) (Merck KGaA); T-Cote® (surface treated with dimethicone) (BASF); Mirasun® TiW60 (surface treated with silica and alumina) (Rhodia); Tayaca MT100T (surface treated with aluminum stearate) (Tayaca); Tayaca MT-100SA (surface treated with silica and alumina) (Tayaca); Tayaca MT-500SA (surface treated with silica and alumina) (Tayaca); Tioveil® EUT, FIN, FLO, FPT, GCM, GPT, IPM, MOTG, OP, TG, TGOP (surface treated with silica and alumina, 40% dispersion in a range of cosmetic vehicle) (ICI); Eusolex® T-45D (surface treated with alumina and simethicone, 45% dispersion in isononoylnonaoate) (Merck KGaA); and Eusolex® T-Aqua (surface treated with aluminum hydroxide, 25% dispersion in water) (Merck KGaA).

Examples of suitable untreated and hydrophobically modified zinc oxide include but are not limited to: Z-Cote® (uncoated microfine zinc oxide) (BASF); Z-Cote® HP-1 (surface treated with dimethicone) (BASF); Sachtotec® LA 10 (surface treated with lauric acid) (Sachtleben); Sachtotec® (uncoated microfine zinc oxide) (Sachtleben); Spectraveil® FIN, IPM, MOTG, OP, TG, TGOP (uncoated, 60% dispersion in a range of cosmetic vehicle) (ICI); Z-sperse® TN (untreated, dispersion in C12-15 alkyl benzoate) (Collaborative); Z-sperse® TN (untreated, dispersion in octydodecyl neopentanoate) (Collaborative).

Most preferably, the skin lightening/even toning compositions of the present invention will comprise a combination of such sunscreen actives. In this respect, it is well known that certain sunscreen actives have better stability, hence longevity, than others; while others have better absorptive capabilities, whether in reference to selectivity for certain UV energy of certain wavelength(s) or cumulative absorptive capabilities. If needed, suitable photostabilizer, for examples, diethylhexyl bezylidene malonates (Oxynex® ST or Oxynex® ST Liquid marketed by EMD/Merck, Germany), 4-methylbenzylidene camphor, butyloctyl salicylate, diethylhexyl 2,6-naphthalate (Corapan® TQ, marketed by Symrise) etc. can also be included to stabilize unstable sunscreen actives. Additionally, synergistic agents may be used in combination with one or more sunscreen compositions including for example bakuchiol. Such synergistic combinations are disclosed in, e.g., the patent application of Ratan Chaudhuri for "Sunscreen Compositions and Methods" filed on May 14, 2007 as Application Serial No. 11/803188. Hence, by using combinations of such UV sunscreen actives, one is able to provide greater prevention of sun-induced hyperpigmentation. Suitable combinations of sunscreen actives are well known in the art and within the skill of a typical artisan in the field.

The skin lightening/even-toning compositions of the present invention may also include one or more skin penetrants. These are additives that, when applied to the skin, have a direct effect on the permeability of the skin barrier: increasing the speed with which and/or the amount by which certain other compounds are able to penetrate into the skin layers. Exemplary organic penetration enhancers include dimethyl sulfoxide; isopropyl myristate; decyl, undecyl or dodecyl alcohol; propylene glycol; polyethylene glycol; $C_{9-11}$, $C_{12-13}$ or $C_{12-15}$ fatty alcohols; azone; alkyl pyrrolidones; diethoxy glycol (Transcutol); lecithin; etc. Surfactants can also be used as penetration enhancers. In the case of hexylresorcinol, penetrants have the benefit of carrying hexylresorcinol into the skin faster than it might otherwise penetrate on its own: thereby expediting and, possible, enhancing the benefit of the hexylresorcinol.

Other optional adjunct ingredients for the skin lightening/even toning compositions of the present invention include preservatives, waterproofing agents, fragrances, anti-foam agents, plant extracts (Aloe vera, witch hazel, cucumber, etc), opacifiers, stabilizers, skin conditioning agents colorants, and the like, each in amounts effective to accomplish their respective functions.

The skin lightening/even toning compositions of the present invention may be prepared by any method known in the art for cosmetic and/or dermatological preparations. Generally, the method comprises the simple mixing of the components; though, especially where insoluble or immiscible components are employed higher agitation or homogenization may be necessary to prepare an appropriate composition, e.g., an emulsion or suspension, etc. Additionally, during the preparation, it may be desirable to add known pH adjusters to in order to maintain a proper pH of the composition for topical application, especially if basic ingredients are to be employed. Generally, the pH should be on the neutral to slightly acidic side, perhaps as low as pH 4. Preferably, though, the pH will be in the range of from about 5 to about 6.5.

The skin lightening/even toning compositions of the present invention are effective in lightening/even toning normal skin, hyperpigmentated skin, as well as skin darkened due to UV exposure. Accordingly, the present invention also pertains to a method of lightening skin and/or providing a more even tone to skin color. Further, the present invention pertains to a method of lightening areas of the skin that have developed hyperpigmentation as a result of physical and/or physiological events including trauma, inflammation, laser therapy, age, diet, drug or pharmaceutical treatment, pregnancy, etc. In addition, the present invention relates to a method of treating hyperpigmentation/skin darkening arising from UV exposure, especially exposure to the sun. Specifically, the method of treating skin to manifest skin lightening and/or more even toning comprises the step of applying the inventive skin whitening composition to the afflicted areas of the skin for so long as necessary to obtain the desired skin lightening effect.

In an alternate respect, the present invention also pertains to a method of preventing or inhibiting hyperpigmentation and/or skin darkening, especially, though not exclusively, that arising from UV exposure. Said method comprises the step of applying the skin lightening compositions of the present invention to those areas of the skin to be affected. For example, areas of inflammation, trauma, laser therapy, etc., may be treated with the novel skin lightening compositions. Alternatively, with respect to prevention of hyperpigmentation arising from short term and/or long term UV exposure, especially exposure to the sun, the method comprises the step of applying the skin lightening compositions to those areas of the skin that are or may be exposed to the sun. It may also be desirable to apply the skin lightening/even toning composition to areas that are not typically exposed to the sun but that nevertheless have exposure to the penetrating UV rays: in this latter respect, it is well known that various articles of clothing have minimal UV blocking capabilities and, hence, skin that is covered by the articles of clothing nevertheless suffer from UV exposure.

The amount of the skin lightening/even toning composition that is to be applied to the skin depends upon the form of the skin lightening/even toning composition and its mode of application. For example, a spray formulation may be applied so as to provide a light, even coat on the skin. Similarly, lotions, creams, gels and the like are typically applied in an amount to provide a light coating to the afflicted or treated area: consistent with the application of topical pharmaceutical ointments, creams, lotions, and the like. Generally speaking, the rate of application, especially where all or substantially all of the skin is to be treated, is about 1 to 2 fluid ounces (29.6 to 59.2 ml) for the entire body, i.e., for the exposed skin of a "average individual" wearing a swimsuit and standing 5 feet 4 inches (1.63 m) tall, weighing 150 pounds (68.3 kg), and having a 32 inch (0.81 m) waist. This translates to an application rate of about 2 mg/cm$^2$ of skin. On the face, a typical application rate is ¼ to 1/3 of a teaspoon (1.2 to 1.7 ml). At such levels of application, the amount of skin lightening agent applied lies in the range of from about 0.1 to about 10 mg/cm$^2$, preferably from about 1 to about 3 mg/cm$^2$, of skin. The composition should be applied to the afflicted areas at least once a day, preferably twice a day.

For those compositions containing sunscreens and, in following, those methods for preventing hyperpigmentation from UV exposure, the skin lightening/even toning composition should be applied before sun exposure, preferably at least 15 minutes before, and reapplied at least every 2 hours or more frequently, especially if the individual engages in activities/actions that may cause the sunscreen containing skin lightening composition to wear or wipe off, e.g., swimming; washing dishes, windows, etc.; washing hands and/or face; contact sports activities; activities that promote substantial sweating; etc.: all actions/events that cause the premature wearing off or loss of the sunscreen containing composition.

## EXAMPLES

Having described the invention in general terms, the following sets of examples will now demonstrate various embodiments of the inventive compositions and their use. In the foregoing and in the following examples, unless otherwise indicated, all temperatures are set forth in degrees Celsius and all parts and percentages are by weight.

**Example 1:** Skin sensitivity

Given the known sensitivity issues associated with commercial grade hexylresorcinol, evaluation of the skin sensitivity to the purified hexylresorcinol was also evaluated. Skin sensitivity was evaluated following the method cited in the reference Appraisal of the Safety of Chemicals in Food, Drugs and Cosmetics, published by The Association of Food and Drug Officials of The United States. The specific method employed used nine inductive patchings, not the ten cited in the reference, under occlusive patch conditions.

Highly pure hexylresorcinol was prepared as follows: A mixture comprising 40 grams hexanoic acid, 6.0 grams zinc chloride and 30 ml of xylene was heated to reflux. 10 grams of resorcinol was gradually added to the foregoing and mixture allowed to reflux for 4 to 6 hours. Water formed during the reaction was removed by the addition of xylene through azeotropic distillation. Thereafter, the reaction mixture was poured into 100 ml of water and the organic layer separated. The separated organic layer was then subjected to fractional distillation to recover the solvent, hexanoic acid and the 4-hexanoylresorcinol. The distilled product was then crystallized from hexane to yield approximately 15 grams crude hexanoylresorcinol. 15 grams hexanoylresorcinol was then dissolved in 30 ml of ethanol and treated with 18 grams of activated zinc and 60 ml of 25% hydrochloric acid at mild reflux. After completion of the reaction the ethanol is removed and the reaction mixture is extracted with toluene. The organic layer is washed with water (30 ml - 2 times) and concentrated to dryness to give a crude hexylresorcinol (yield 13gm). This on crystallization from hexane gave 10 grams of hexylresorcinol having resorcinol content of 0.005% to nil.

Samples were prepared for evaluation by diluting the highly pure hexylresorcinol in corn oil to a 5% concentration, with dilutions freshly prepared on each application day. 0.2 ml of the diluted test material was dispensed onto occlusive, hypoallergenic patches and the treated patches applied directly to the skin of the infrascapular regions of the back, to the right or left of the midline of each subject: one hundred and eleven subjects were employed. After application of the patch, each subject was dismissed with instructions not to wet or expose the test area to direct sunlight. The patch was removed by the subject after 24 hours. This procedure was repeated every Monday, Wednesday and Friday for three consecutive weeks until a series of nine consecutive 24 hour exposures had been made. During the test period, the test area on the subjects' backs were observed for evidence of edema or erythema just before applications two through nine and the next test date following application nine. If evidence of a reaction was found, the area of edema and/or erythema was then measured and recorded: edema being estimated by an evaluation of the skin with respect to the contour of the unaffected normal skin. The subjects were then given a 10 - 14 day rest period after which a challenge or retest patch saturated with 0.2 ml of the hexylresorcinol dilutions was applied to a previously unexposed test site. The challenge or retest sites were monitored and scored 24 and 48 hours after application. Based on the test results, the 5% dilution in corn oil of the purified hexylresorcinol was determined to be a NON-PRIMARY IRRITANT and a NON-PRIMARY SENSITIZER according to the reference.

**Example 2/Comparative Example 1 -** Skin lightening efficacy

A skin lightening composition was prepared having the formula set forth in Table 2. The composition was prepared by mixing the components of Phase A-1 until a substantially homogenous mixture was obtained and then dispersing that mixture in Phase A-2 with moderate agitation. The mixture was mixed until a homogeneous clear dispersion was obtained. Then, the components of Phase B were combined, mixed and heated to 75°C and then added to the Phase A-1/A-2 mixture. The so formed mixture was homogenized for 2-3 minutes to obtain a substantially uniform composition. Phase C was added and mixed until uniform. After cooling down to 50°C, Phases D, E and F in the listed order were added and mixed and the final composition then cooled down to room temperature. The final skin lightening composition was found to have a pH value of 6.2 at 25°C. A second skin lightening composition was prepared in the same manner with the same formulation except that the highly purified hexylresorcinol was replaced with 2% hydroquinone (Eastman) and water adjusted accordingly.

Each of the skin lightening compositions was applied to different test sites on the forearms of fifteen individuals. The compositions were applied twice a day, morning and evening, for eight weeks. The test sites as well as untreated sites on each forearm were evaluated by a trained clinical evaluator for brightness of skin, evenness of skin tone, appearance of skin, dryness of skin and radiance of skin using a 10 point scale prior to the first application and following Week 4 and Week 8. Each test site was evaluated for skin color change as represented by the change in L values and ITA.degree (Individual Topology Angle - COLIPA SPF test method) as measured by chromometric measurement. ITA.degree. was calculated using the formula:

$$ITA.degree. = [Arc\ Tangent(L*-50)/b*)]180/3.1416$$

Wherein, L*value - lightness and b* - color in blue-yellow axis. The results of the testing were as presented in Table 3, wherein the delta represents the percent change in skin color from the baseline coloration of the untreated skin.

Table 2 - Skin Lightening Composition

| Ingredient | Trade Name / Supplier | Wt % |
|---|---|---|
| **Phase A-1** | | |
| Water (demineralized) | | 74.70 |
| Disodium EDTA | | 0.10 |

(continued)

| Ingredient | Trade Name / Supplier | Wt % |
|---|---|---|
| Glycerin | | 3.00 |
| **Phase A-2** | | |
| Acrylates/C 10-30 Alkyl Acrylate Copolymer | Carbopol Ultrez 21BF Goodrich | 0.20 |
| **Phase B** | | |
| Caprylic/Capric Triglycerides | Myritol 318/Cognis | 4.00 |
| Cetearyl Isononaoate | Cetiol SN/Cognis | 4.00 |
| Glyceryl Stearate, PEG-100 Stearate | Arlacel 165/Uniquema | 2.00 |
| Sorbitan Stearate | Arlacel 60/Uniquema | 0.50 |
| Dimethicone | DC, 200/100CST | 1.00 |
| Gransil GCM | Cyclomethicone, Polysilicone-11, Petrolatum/Grant Industries | 5.00 |
| Cetyl Esters | Crodamol SS/Croda | 1.00 |
| **Phase C** | | |
| Hydroxyethyl Acrylate/Sodium Acryloyldimethyl Taurate Copolymer/Squalane/Polysorbate 60 | Simugel NS/Seppic | 0.75 |
| **Phase D** | | |
| Ethoxydiglycol | Transcutol/Gattefosse | 2.00 |
| **Hexylresorcinol** | **Synovea™ HR/Sytheon\*** | **0.50** |
| **Phase E** | | |
| AMP-95 (aminomethyl propanol) | to pH- 6.20 | 0.25 |
| **Phase F** | | |
| Phenoxyethanol, Methylparaben, Propylparaben | Phenonip XB/Clariant | 1.00 |
| Total | <u>100.00</u> | <u>100.00</u> |
| * Synovea™ HR hexylresorcinol available from Sytheon Ltd. of Lincoln Park, NJ 07035) | | |

As seen from Table 3, even a 0.5% purified hexylresorcinol composition provided a comparable, statistically significant, change in skin color to that attained with like composition containing 2% hydroquinone: perhaps the most effective known skin whitening agent. Such results are consistent with a "lightening" of the skin. No adverse effects were noted for either composition over the short test period.

**TABLE 3**

| Skin Lightening Active | Week | $\Delta$L Value | $\Delta$ITA Degrees |
|---|---|---|---|
| Hexylresorcinol - 0.5% | 4 | 3.4 | 27.48 |
| | 8 | 4.67 | 37.56 |
| Hydroquinone - 2% | 4 | 3.24 | 27.66 |
| | 8 | 4.27 | 39.38 |

**Example 3:** Skin lightening formulations 3A - 3K

The following tables set forth various formulations and embodiments of skin lighteners/even toners according to the present invention. In each case the highly pure hexylresorcinol employed was Synovea™ HR hexylresorcinol available from Sytheon Ltd. of Lincoln Park, NJ 07035. Following each table is a brief description of the process by which each formulation is made.

Formulation 3A: Skin lightening lotion

| Ingredient | Trade Name / Supplier | Wt % |
|---|---|---|
| **Phase A** | | |
| Water (demineralized) | | 56.15 |
| Disodium EDTA | | 0.10 |
| Propylene Glycol | | 2.00 |
| Sorbitol | Sorbo (70% soln.)/Uniqema | 2.00 |
| Sodium Lauryl Sulfate | Stepanol ME-Dry/Stepan | 0.15 |
| **Phase B** | | |
| Glyceryl Stearate | Tegin M/Goldschmidt | 5.00 |
| Stearic acid | Emersol 132/Cognis | 1.00 |
| Persea Gratissima (Avocado) oil Unsaponifiables | Crodarom Avocadin/Croda | 15.00 |
| Beeswax | White Bleached NF Beeswax Prills/Ross | 1.50 |
| **Phase C** | | |
| **Highly purified Hexylresorcinol** | Synovea HR / Sytheon Ltd | 1.00 |
| Ethoxydiglycol | Transcutol / Gattefose | 4.00 |
| **Phase D** | | |
| Phyllanthus emblica fruit extract (skin lightener, antioxidant, wrinkle reducer) | Emblica / EMD Chemicals | 1.00 |
| Water | | 10.00 |
| **Phase E** | | |
| Triethanolamine | TEA 99%/Union Carbide | 0.10 |
| **Phase F** | | |
| Propylene glycol, DMDM Hydantoin, Methylparaben | Paragon/McIntyre | 1.00 |
| **Total** | | 100.00 |

Formulation 3A is prepared by separately combining the ingredients of Phases A and B and heating each mixture to 70-75°C. Thereafter, Phases A and B are combined while stirring. After cooling to about 40-50 °C, Phase C is added to the mixture of Phases A and B. Subsequently, Phase D is added with mixing until a uniform, substantially homogenous mixture is attained. The pH is then adjusted 5 to 5.5 by adding Phase E and then Phase F is added with mixing until uniform.

Formulation 3B - Daily skin brightening lotion

| Ingredient | Trade Name / Supplier | Wt% |
|---|---|---|
| **Phase A-1** | | |
| Water (demineralized) | | 64.18 |
| Disodium EDTA | | 0.05 |
| Propylene Glycol | | 5.00 |
| **Phase A-2** | | |
| Xantham Gum | Vanzan NF/Vanderbilt | 0.25 |
| Magnesium aluminum stearate | Veegum Ultra granules/Vanderbilt | 0.40 |
| **Phase B** | | |
| Cetearyl alcohol and cetearyl glucoside | Montanov 68/Seppic | 7.00 |
| Apricot kernel oil | Lipovol P/Liop | 10.00 |
| Octyl stearate | Cetiol 868/Cognis | 3.00 |

(continued)

| Ingredient | Trade Name / Supplier | Wt% |
|---|---|---|
| Dimethicone | Dow Corning 200 fluid 10cst/Dow Corning | 6.00 |
| **Phase C** | | |
| **Highly purified Hexylresorcinol** | **Synovea™ HR / Ltd** | **1.00** |
| Propylene glycol | | 1.00 5.00 |
| **Phase D** | | |
| Niacinamide (skin lightener, moisturizer) | | 2.00 |
| **Phase E** | | |
| Phenoxyethanol, Isopropylparaben, Isobutylparaben, butylparaben | Liquapar PE/Sutton | 1.00 |
| Total | | 100.00 |

Formulation 3B is prepared by separately combining the ingredients of each of Phases A-1 and A-2 and then dispersing Phase A-2 in Phase A-1 and heating to 70-75°C. The mixture of Phase B is then heated to 70-75°C and added to the Phase A dispersion with constant stirring. The mixture is homogenized until it cools to 50°C. Thereafter Phase C is added and continually mixed. Then Phases D and E are added sequentially and the mixture continually mixed until uniform.

Formulation 3C: Photostable Broad-Spectrum Sunscreen (SPF -20) with Skin Brighteners

| Ingredient | Trade Name/Supplier | Wt % |
|---|---|---|
| **Phase A-1** | | |
| Deionized water | | 59.95 |
| Disodium EDTA | | 0.10 |
| Propylene glycol | Propylene glycol / Lyondell | 3.00 |
| Glycerin | | 2.00 |
| **Phase A-2** | | |
| Acrylates/C10-30 Alkyl Acrylates cross polymer | | 0.15 |
| Xanthan gum | Vanzan NF / Vanderbilt | 0.15 |
| **Phase B** | | |
| Cetyl Alcohol, Glyceryl Stearate, PEG-75, Ceteth-20 and Steareth-20 | Emolium Delta / Gattefosse | 4.00 |
| Dimethicone | DC 200 fluid, 100 cs /Dow Corning | 0.50 |
| C30-38 Olefin/isopropyl Maleate/MA Coplymer | Performa V1608 / New Phase | 1.00 |
| C12-15 Alkyl Benzoate | Finsolv TN / Finetex | 10.00 |
| Butyl Methoxydibenzoylmethane (sunscreen) | Eusolex 9020 / EMD Chemicals | 1.00 |
| Diethylhexyl Syringylidene Malonate (photostabilizer), Caprylic/Capric Triglycerides | Oxynex ST Liquid / EMD Chemicals | 1.00 |
| Homosalate (sunscreen) | Eusolex HMS / EMD Chemicals | 8.00 |
| **Phase C** | | |
| Ethoxydiglycol | Transcutol / Gattefosse | 2.00 |

(continued)

| Ingredient | Trade Name/Supplier | Wt % |
|---|---|---|
| **Highly purified Hexylresorcinol** | Synovea™ HR / Sytheon Ltd | 0.50 |
| **Phase D** | | |
| *Phyllanthus emblica* fruit extract (skin lightener, antioxidant, wrinkle reducer) | Emblica / EMD Chemicals | 0.50 |
| Deionized water | | 5.00 |
| **Phase E** | | |
| Aminoethylpropanol amine | | 0.15 |
| **Phase F** | | |
| Phenoxyethanol, Methy paraben, Ethyl paraben and Propyl paraben | Phenonip XB | 1.00 |
| **Total** | | 100 |

Formulation 3C is prepared by separately combining the ingredients of each of Phases A-1 and A-2 and then dispersing Phase A-2 in Phase A-1 and heating to 70-75°C. The mixture of Phase B is then heated to 70-75°C and added to the Phase A dispersion with constant stirring. The mixture is homogenized until it cools to 50°C. Thereafter Phase C is added and continually mixed. Then Phases D, E and F are sequentially added and the mixture continually mixed until uniform. pH was found to be 5.3.

Formulation 3D: Skin lightening lotion with broad-spectrum sunscreen actives

| Ingredient | Trade Name | Wt % |
|---|---|---|
| **Phase A** | | |
| Butyl Methoxydibenzoylmethane (sunscreen) | Eusolex 9020 | 1.00 |
| Glyceryl Stearate, Cetareth-15 | Tego Care 215, Pellets | 3.00 |
| Decyl Oleate | Cetiol V | 5.00 |
| Isopropyl Palmitate | | 5.00 |
| Dimethicone | Mirasil DM 350 | 0.50 |
| Stearyl Alcohol | Lanette 18 | 2.00 |
| Carbomer | Carbopol ETD 2050 | 0.10 |
| **Phase B** | | |
| Glycerin (about 87%) | Glycerol | 3.00 |
| Ectoin (moisturizer, skin protectant) | RonaCare Ectoin | 0.50 |
| Preservative | | 1.00 |
| Water, Ethyhexyl methoxycinnamate (sunscreen), Silica, PVP, Chlorphenesin, BHT | Eusolex UV-Pearls OMC | 15.00 |
| Water | Water, deminaralized | 60.85 |
| **Phase C** | | |
| **Highly purified Hexylresorcinol** | Synovea™ HR / Sytheon Ltd. | 0.50 |
| Butylene glycol | | 2.00 |
| **Phase D** | | |
| Sodium hydroxide | Sodium hydroxide, 10% solution | 0.45 |
| **Phase E** | | |
| Perfume | Fragrance "Delicat" | 0.20 |
| Total | | 100.00 |

Formulation 3E is prepared by separately combining the ingredients of each of Phase A and Phase B and heating each mixture to 80°C. Phases A and B are then combined with constant stirring. The combined mix is homogenized until the mixture cools to 60°C. Phase C is then added at 40°C. The pH is then adjusted to 5.0-6.0 with phase D. Thereafter, Phase E is added and mixed until a uniform mixture is attained.

Formulation 3F: Anhydrous Oil-Free skin lightening gel

| Ingredient | Trade Name / Supplier | Wt % |
|---|---|---|
| **Phase A** | | |
| Ozokerite | White Ozokerite SP-1020/Strahl & Pitsch | 3.00 |
| Cyclomethicone | Dow Corning 345 Fluid/Dow Corning | 25.00 |
| Cyclomethicone (and) Polysilicone-11 | Gransil GCM/Grant Industries | 60.00 |
| **Phase B**<br>Bismuth Oxychloride<br>**Phase C**<br>Cyclomethicone | Biron® LF-2000/Rona<br><br>Dow Corning 345 Fluid/Dow Corning | 2.00<br><br>3.10 |
| Cyclomethicone (and) Dimethicone Crosspolymer | Dow Corning 9040 Silicone Elastomer Blend/Dow Corning | 5.40 |
| Bakuchiol (antioxidant, anti-inflammatory and anti-bacterial) | Sytenol A / Sytheon Ltd | 1.00 |
| **Highly purified Hexylresorcinol** | **Synovea™ HR / Sytheon Ltd** | 0.50 |
| **Total** | | 100.00 |

Formulation 3F is prepared by blending the Phase A ingredients while heating to 70-75°C and mixing until clear and uniform mixture is obtained. Phase B is then dispersed in the Phase A mixture with mixing. The Phase C ingredients are separately blended until the mixture is smooth and substantially free of lumps. The Phase A/B mixture is then cooled to 50 - 60° C and Phase C added with mixing until a substantially uniform mixture is obtained.

Formulation 3G: Sunscreen Cream with skin lightener

| Ingredient | Trade Name/Supplier | Wt % |
|---|---|---|
| **Phase A** | | |
| Titanium Dioxide, Alumina, Simethicone | Eusolex® T-2000/EMD | 10.00 |
| Polyglyceryl-2 Dipolyhydroxystearate | Dehymuls PGPH/Cognis | 4.00 |
| Polyglyceryl-3 Diisostearate | Lamaform TGI FL/Cognis | 2.00 |
| Beeswax | Beeswax White SP 422/Strahl & Pitsch | 3.00 |
| Isostearic Acid | Emersol 871/Cognis | 1.00 |
| Zinc Stearate | Unichem ZS/Universal Preserv-A-Chem | 1.00 |
| Dicaprylyl Carbonate | Cetiol CC/Cognis | 11.00 |
| Tocopherol (antioxidant) | Vitamin E/Hoffmann- La Roche | 2.00 |
| **Highly purified Hexylresorcinol** | **Synovea™ HR / Sytheon ltd.** | **1.00** |
| Propylparaben | Nipasol M/Clariant | 0.05 |
| **Phase B** | | |
| Water (demineralized) | | 48.30 |
| Magnesium Sulfate | Magnesium Sulfate Heptahydrate/Rona | 1.00 |
| Methylparaben | Nipagin M/Clariant | 0.15 |
| Glycerin | Emery 916/Cognis | 5.00 |
| **Phase C** | | |

(continued)

| Ingredient | Trade Name/Supplier | Wt % |
|---|---|---|
| Bisabolol (anti-inflammatory) | RonaCare® Bisabolol/EMD | 0.50 |
| Total | | 100.00 |

Formulation 3G is prepared by separately combining the ingredients of Phase A and Phase B and heating each mixture to 80°C. Phase B is then added slowly to phase A while stirring. The mixture is homogenized at 65-55°C and then cooled while stirring. Once the temperature reaches 40°C, Phase C is added and the mixture mixed until uniform. The mixture has a viscosity of about 80,000 mPas (Brookfield RVT) at 24 C.

Formulation 3H: Broad Spectrum Sunscreen Lotion with skin lightener

| Ingredient | Trade Name/Supplier | Wt % |
|---|---|---|
| **Phase A-1** | | |
| Deionized water | | 60.10 |
| Disodium EDTA | | 0.10 |
| Proylene Glycol | | 3.00 |
| Glycerin | | 2.00 |
| **Phase A-2** | | |
| Acrylates/C10-30 Alkyl Acrylate Copolymer | Carbopol EDT 2020/Goodrich | 0.15 |
| Xanthan Gum | Vanzan NF/Vanderbilt | 0.15 |
| **Phase B** | | |
| Cetyl alcohol, glyceryl stearate, PEG-75, ceteth-20 and steareth-20 | Emolium Delta / Gattefosse | 4.00 |
| Bakuchiol | Sytenol A / Sytheon Ltd | 1.00 |
| Dimethicone | DC200 fluid, 100cst/Dow | 0.50 |
| C30-38 Olefin/Isopropyl Maleate / MA Copolymer | Performa V1608 / New Phase Technologies | 1.00 |
| C12-15 Alkyl benzoate | Finsolv TN / Finetex | 10.00 |
| Butyl methoxydibenzoylmethane (sunscreen) | Eusolex 9020 / EMD | 2.00 |
| Diethylhexyl syringylidene malonate (photostabilizer) | Oxynex ST / EMD | 2.00 |
| Homosalate (sunscreen) | Eusolex HMS/EMD | 8.00 |
| **Phase C** | | |
| **Highly purified Hexylresorcinol** | Synovea™ HR / Sytheon Ltd. | 0.50 |
| Laureth-23 | Lipocol L-23/Lipo | 0.50 |
| Methyl Gluceth-20 | Glucam E-20/Noveon | 4.50 |
| **Phase D** | | |
| Phenoxyethanol (and) Isopropylparaben (and) Isobutylparaben (and) Butylparaben | Liquapar PE/ISP | 1.00 |
| **Total** | | 100.00 |

Formulation 3H is prepared by combining the ingredients of Phase A-1 and then dispersing Phase A-2 in the Phase A-1 mixture with agitation and heating the combination to 75°C. Separately, the Phase B ingredients are combined and heated to 75°C. The Phase B mixture is then added to the Phase A-1/A-2 combination with continuous stirring. The mixture is homogenized for 10 min and cooled to 45°C. Phases C and D are then sequentially added and mixed until uniform.

Formulation 3I: Night Skin Brightener for normal skin

| INCI Name | Trade Name/Supplier | % w/w |
|---|---|---|
| **Phase A-1** | | |
| Deionized water | | 55.65 |
| Disodium EDTA | | 0.10 |
| Propylene glycol | Propylene glycol / Lyondell | 2.00 |
| Xylitol | | 3.00 |
| **Phase A-2** | | |
| Xanthan gum | Vanzan NF / Vanderbilt | 0.20 |
| **Phase B** | | |
| PEG-6 Stearate, Ceteth-20, Glyceryl Stearate, Stearath-20, Stearic Acid | Tefose 2561 / Gattefosse | 8.00 |
| Hydrogenated castor oil | Cutina HR / Cognis | 1.00 |
| Octyldodecyl Myristate | M.O.D. / Gattefose | 8.00 |
| Dimethicone | Dow Corning 200, 50 cst / Dow Corning | 3.00 |
| Phenyltrimethicone | Dow Corning 556 Wax / Dow Corning | 1.00 |
| Sweet Almond oil | Cropure Almond / Croda | 3.00 |
| Licorice extract (skin lightener) | Pentapharm | 0.10 |
| **Phase C** | | |
| Ethoxydiglycol | Tarnscutol / Gattefosse | 2.50 |
| **Highly purified Hexylresorcinol** | **Synovea HR / Sytheon Ltd** | 0.75 |
| **Phase D** | | |
| *Phyllanthus emblica* fruit extract | Emblica / EMD Chemicals | 1.00 |
| Deionized water | | 10.00 |
| **Phase E** | | |
| Aminoethylpropanol amine or Triethanol amine (99%) | | 0.15 |
| **Phase F** | | |
| Propylene Glycol, DMDM Hydantoin, Methyl paaraben, Propylparaben | Paragon / McIntyre | 1.00 |
| **Total** | | **100.00** |

Formulation 3I is prepared by combining the ingredients of Phase A-1 and then dispersing Phase A-2 in the Phase A-1 mixture with agitation and heating the combination to 75°C. Separately, the Phase B ingredients are combined and heated to 75°C. The Phase B mixture is then added to the Phase A combination with continuous stirring. The mixture is homogenized for 10 min and cooled to 45°C. Phases C and D are then sequentially added and mixed until uniform.

This formulated product 3I was applied twice a day to five subjects with hyperpigmented spots caused by sun light for a period of 4 weeks. Comparison of the initial intensity of hyperpigmentation spot vs treated site showed significant lightening (visually) as judged by the subjects as well as by the technician. A similarly formulated product, without the highly purified hexylresorcinol but with the skin lightener *Phyllanthus emblica* fruit extract, did not show any perceivable difference in reducing hyperpigmented spots of five patients evaluated even after 8 weeks.

Formulation 3J: Anhydrous skin lightening lotion for reducing scar-induced hyperpigmentation

| Ingredient | Trade Name/Supplier | Wt % |
|---|---|---|
| **Phase A** | | |
| Cyclomethicone, Polysilicone-11 | Gransil GCM-5 / Gransil | 49.50 |
| Dimethicone ( | Dow Corning 345 / DC (200 fluid, 10 cst) | 25.00 |
| **Phase B** | | |
| Silica | Spheron P -1500 / Presperse | 2.00 |
| Isohexadecane | Permethyl 101 / Presperse | 5.00 |
| **Phase C** | | |
| Diethoxydiglycol | Transcutol CG | 5.00 |
| **Highly purified Hexylresorcinol** | Synovea™ HR / Sytheon | 0.50 |
| **Phase D** | | |
| Polyethylene | | 1.50 |
| Petrolatum | | 1.50 |
| Isohexadecane | Permethyl 101 / Presperse | 10.00 |
| **Total** | | 100.00 |

Formulation 3J is prepared by combining the ingredients of Phase A and then dispersing Phase B in the Phase A mixture with agitation at room temperature. Separately, the Phase C ingredients are combined and mixed well until uniform solution is obtained. The Phase C mixture is then added to the Phases A and B combination and homogenized for 10 min. Phase D is then added and mixed until uniform. The viscosity of this formulation was found to be 30,000 cps (Brookefield RVT, Spindle C, Helipath) at 25 C.

This formulated product 3J was applied twice a day to five subjects with hyperpigmented spots caused by laser therapy for a period of 4 weeks. Comparison of the initial intensity of hyperpigmentation spot vs treated site showed significant lightening (visually) as judged by the subjects as well by the technician. Similar formulated product without the highly purified hexylresorcinol did not show any perceivable difference in laser therapy-induced hyperpigmented spots of five patients evaluated even after 8 weeks.

Formulation 3K: Skin Brightening Serum for dark circle treatment

| INCI Name | Trade Name / Supplier | % w/w |
|---|---|---|
| **Phase A** | | |
| Water (demineralized) | | 90.10 |
| Glycerin or Xylitol | | 2.00 |
| Ascorbyl glucoside (skin lightener) | | 2.00 |
| **Phase B** | | |
| Hydroxypropyl Methylcellulose | Benecel MP 333C/Hercules | 0.30 |
| Sodium Hyaluronate | Rita HA C-1-P/Rita | 0.30 |
| **Phase C** | | |
| **Highly Purified Hexylresorcinol** | **Synovea™ HR/Sytheon** Ltd. | 0.50 |
| Laureth-23 | Lipocol L-23/Lipo | 0.50 |
| Methyl Gluceth-20 | Glucam E-20/Noveon | 3.50 |
| **Phase D** | | |
| Phenoxyethanol | Phenoxyethanol/Clariant | 0.80 |

(continued)

| INCI Name | Trade Name / Supplier | % w/w |
|---|---|---|
| Total | | 100.00 |

Formulation 3K is prepared by dispersing the combined ingredients of Phase B in the combined ingredients of the Phase A with moderate agitation and mixed until a homogeneous clear gel is obtained. Premixed Phase C is added followed by Phase D under constant stirring until a uniform mixture is attained. pH value of the formulation was found to be 5.40 at 25°C.

This formulated product 3K was applied twice a day to five subjects having dark circle around the eyes for a period of 4 weeks. Comparison of the initial intensity of dark circle vs treated site showed significant lightening as judged by the subjects as well by the technician. A similarly formulated product but without highly purified hexylresocinol did not manifest any difference in the dark circle around the eyes of five patients evaluated even after 8 weeks.

Many of the formulation set forth above contain ingredients other than the critical ingredients including surfactants, stabilizers, antioxidant and the like. These additional ingredients could easily have been omitted without compromising the skin lightening/even toning properties thereof.

Without further elaboration, it is believed that one skilled in the art, using the preceding description, will be sufficiently enabled to utilize the present invention to its fullest extent. Furthermore, while the present invention has been described with respect to the aforementioned specific embodiments and examples, it should be appreciated that other embodiments and extensions thereof based upon and utilizing the concepts of the present invention are possible.

## Claims

1. A stable skin lightening/even toning composition for preventing or lessening pigmentation of normal human skin or of hyper-pigmented human skin, said composition comprising (i) a skin lightening effective amount of highly purified hexylresorcinol, (ii) optionally, from about 0.01 to about 20 weight percent of at least one other skin lightening agent, and (iii) a dermatologically acceptable carrier, wherein said hexylresorcinol has a purity of at least 99 wt % and a resorcinol content, if present, of no greater than 0.1 wt %.

2. The skin lightening/even toning composition of claim 1 wherein the said highly purified hexylresorcinol is present in an amount of from about 0.05 to about 10 percent by weight.

3. The skin lightening/even toning composition of claim 1 or 2 wherein the second skin lightening agent is present and the weight ratio of the highly purified hexylresorcinol to the second skin lightening agent is from 20: 1 to about 1:20.

4. The skin lightening/even toning composition of claim 1, 2 or 3 wherein the highly purified hexylresorcinol is present in an amount of from about 0.1 to about 5 wt %, the second skin lightening agent is present in an amount of from about 0.1 to about 5 wt %, and the weight ratio of the highly purified hexylresorcinol to the second skin lightening agent is from about 10:1 to about 1:10.

5. The skin lightening/even toning composition of any of claims 1 to 4 wherein the highly purified hexylresorcinol contains 0.005 wt% to nil of resorcinol and has a purity of at least 99 wt%.

6. The skin lightening/even toning composition of any of claims 1 to 5 wherein the high purity hexylresorcinol has been isolated from crude hexylresorcinol by extraction with toluene and crystallization from hexane.

7. The skin lightening/even toning composition of any of claims 1 to 6 further comprising one or more conventional skin protective or treatment ingredients selected from the group consisting of sunscreen actives, antioxidants, vitamins, anti-inflammatory agents, moisturizers, emollients, humectants, and mixtures thereof in an effective amount.

8. The skin lightening/even toning composition of any of claims 1 to 7 wherein the second skin lightening agent is present and the skin lightening efficacy of the composition is greater than for a like composition containing just the second skin lightening agent in amount equal to the combined amount of hexylresorcinol and second skin lightening agent.

9. Use of a skin lightening/even toning composition comprising (i) a skin lightening effective amount of highly purified hexylresorcinol, (ii) optionally, from about 0.01 to about 20 weight percent of at least one other skin lightening agent, and (iii) a dermatologically acceptable carrier, wherein the said highly purified hexylresorcinol has a purity of at least 99 wt % and a resorcinol content, if present, of no greater than 0.1 wt %. in a cosmetic method of lightening skin color and/or providing a more even tone to skin color.

10. The use of claim 9 wherein the skin lightening/even toning composition is applied to the areas to be lightened in an amount sufficient to provide a light coating to the skin at least twice a day until the desired skin lightening effect is achieved.

11. The use of any of claims 9 or 10 wherein the composition is applied to areas of the skin to be protected from darkening due to age, diet or pregnancy.

12. Skin lightening/even toning composition according to any of claims 1-8 for use on areas of the skin to be protected from skin darkening due to UV light, sun exposure, and physical and/or physiological conditions selected from trauma, inflammation, laser therapy, drug or pharmaceutical treatment.

**Patentansprüche**

1. Eine stabile hautaufhellende/gleichmäßig tönende Zusammensetzung zur Verhinderung oder Verringerung der Pigmentierung von normaler menschlicher Haut oder von hyperpigmentierter menschlicher Haut, wobei die Zusammensetzung (i) eine wirksame hautaufliellende Menge von hochreinem Hexylresorcinol, (ii) gegebenenfalls etwa 0,01 bis etwa 20 Gewichtsprozent mindestens eines weiteren hautaufhellenden Mittels und (iii) einen dermatologisch verträglichen Träger umfasst, wobei das Hexylresorcinol eine Reinheit von mindestens 99 Gew.-% und einen Resorcinolgehalt, falls vorhanden, von nicht größer als 0,1 Gew.-% aufweist.

2. Die hautaufhellende/gleichmäßig tönende Zusammensetzung gemäß Anspruch 1, wobei das hochreine Hexylresorcinol in einer Menge von etwa 0,05 bis etwa 10 Gewichtsprozent vorhanden ist.

3. Die hautaufhellende/gleichmäßig tönende Zusammensetzung gemäß Anspruch 1 oder 2, wobei das zweite hautaufhellende Mittel vorhanden ist und das Gewichtsverhältnis des hochreinen Hexylresorcinols zum zweiten hautaufhellenden Mittel von 20:1 bis etwa 1 20 beträgt.

4. Die hautaufhellende/gleichmäßig tönende Zusammensetzung gemäß Anspruch 1, 2 oder 3, wobei das hochreine Hexylresorcinol in einer Menge von etwa 0,1 bis etwa 5 Gew.-% vorhanden ist, das zweite hautaufhellende Mittel in einer Menge von etwa 0,1 bis etwa 5 Gew.-% vorhanden ist und das Gewichtsverhältnis des hochreinen Hexylresorcinols zum zweiten hautaufhellenden Mittel etwa 10:1 bis etwa 1:10 beträgt.

5. Die hautaufhellende/gleichmäßig tönende Zusammensetzung gemäß einem der Ansprüche 1 bis 4, wobei das hochreine Hexylresorcinol 0,005 Gew.-% bis null an Resorcinol enthält und eine Reinheit von mindestens 99 Gew.-% hat.

6. Die hautaufhellende/gleichmäßig tönende Zusammensetzung gemäß einem der Ansprüche 1 bis 5, wobei das Hexylresorcinol mit hoher Reinheit aus rohem Hexylresorcinol durch Extraktion mit Toluol und Kristallisation aus Hexan isoliert wurde.

7. Die hautaufhellende/gleichmäßig tönende Zusammensetzung gemäß einem der Ansprüche 1 bis 6, weiter umfassend ein oder mehrere gebräuchliche Inhaltsstoffe zum Schutz oder zur Behandlung der Haut, ausgewählt aus der Gruppe bestehend aus Sonnenschutzwirkstoffen, Antioxidantien, Vitaminen, entzündungshemmenden Mitteln, Befeuchtungsmitteln, Emollientia, Feuchthaltemitteln, und Gemischen davon in einer wirksamen Menge.

8. Die hautaufhellende/gleichmäßig tönende Zusammensetzung gemäß einem der Ansprüche 1 bis 7, wobei das zweite hautaufhellende Mittel vorhanden ist und die hautaufhellende Wirksamkeit der Zusammensetzung größer ist als für eine vergleichbare Zusammensetzung, welche lediglich das zweite hautaufhellende Mittel in einer Menge gleich der kombinierten Menge an Hexylresorcinol und dem zweiten hautaufhellenden Mittel umfasst.

9. Verwendung einer hautaufhellenden/gleichmäßig tönenden Zusammensetzung, umfassend (i) eine wirksame hautaufhellende Menge von hochreinem Hexylresorcinol, (ii) gegebenenfalls etwa 0,01 bis etwa 20 Gewichtsprozent mindestens eines weiteren hautaufhellenden Mittels und (iii) einen dermatologisch verträglichen Träger, wobei das hochreine Hexylresorcinol eine Reinheit von mindestens 99 Gew.-% und einen Resorcinolgehalt, falls vorhanden, von nicht größer als 0,1 Gew.-% aufweist, in einem kosmetischen Verfahren zur Aufhellung der Hautfarbe und/oder zur Bereitstellung einer gleichmäßigeren Tönung der Hautfarbe.

10. Die Verwendung gemäß Anspruch 9, wobei die hautaufhellende/gleichmäßig tönende Zusammensetzung in einer Menge, die ausreichend ist, um eine leichte Bedeckung der Haut bereitzustellen, mindestens zweimal pro Tag auf die aufzuhellenden Bereiche aufgetragen wird, bis der gewünschte hautaufhellende Effekt erreicht ist.

11. Die Verwendung gemäß einem der Ansprüche 9 oder 10, wobei die Zusammensetzung auf Bereiche der Haut, welche vor Dunklung aufgrund Alterung, Ernährung oder Schwangerschaft geschützt werden sollen, aufgetragen wird.

12. Hautaufhellende/gleichmäßig tönende Zusammensetzung gemäß einem der Ansprüche 1 bis 8 zur Verwendung auf Bereichen der Haut, welche vor Hautdunklung aufgrund UV-Licht, Sonnenexposition und physischen und/oder physiologischen Bedingungen, ausgewählt aus Trauma, Entzündung, Lasertherapie, medikamentöser oder pharmazeutischer Behandlung, geschützt werden soll.

## Revendications

1. Composition tonifiante éclaircissante/uniformisante stable pour la peau destinée à la prévention ou à la réduction de la pigmentation d'un peau humaine normale ou d'une peau humaine hyper-pigmentée, ladite composition comprenant (i) une quantité efficace pour l'éclaircissement de la peau d'hexylrésorcinol hautement purifié, (ii) facultativement d'environ 0,01 à environ 20 % en poids d'au moins un autre agent d'éclaircissement de la peau, et (iii) un véhicule acceptable d'un point de vue dermatologique, dans laquelle ledit hexylrésorcinol a une pureté d'au moins 99 % en poids et une teneur en résorcinol, le cas échéant, non supérieure à 0,1 % en poids.

2. Composition tonifiante éclaircissante/uniformisante pour la peau selon la revendication 1, dans laquelle ledit hexylrésorcinol hautement purifié est présent en une quantité d'environ 0,05 à environ 10 % en poids.

3. Composition tonifiante éclaircissante/uniformisante pour la peau selon la revendication 1 ou 2, dans laquelle le second agent d'éclaircissement de la peau est présent et le rapport pondéral de l'hexylrésorcinol hautement purifié au second agent d'éclaircissement de la peau est de 20:1 à environ 1:20.

4. Composition tonifiante éclaircissante/uniformisante pour la peau selon la revendication 1, 2 ou 3, dans laquelle l'hexylrésorcinol hautement purifié est présent en une quantité d'environ 0,1 à environ 5 % en poids, le second agent d'éclaircissement de la peau est présent en une quantité d'environ 0,1 à environ 5 % en poids et le rapport pondéral de l'hexylrésorcinol hautement purifié au second agent d'éclaircissement de la peau est d'environ 10:1 à environ 1:10.

5. Composition tonifiante éclaircissante/uniformisante pour la peau selon l'une quelconque des revendications 1 à 4, dans laquelle l'hexylrésorcinol hautement purifié contient de 0,005 % en poids à pas de résorcinol et a une pureté d'au moins 99 % en poids.

6. Composition tonifiante éclaircissante/uniformisante pour la peau selon l'une quelconque des revendications 1 à 5, dans laquelle l'hexylrésorcinol hautement purifié est isolé à partir d'hexylrésorcinol brut par extraction avec du toluène et cristallisation à partir d'hexane.

7. Composition tonifiante éclaircissante/uniformisante pour la peau selon l'une quelconque des revendications 1 à 6, comprenant en outre un ou plusieurs ingrédients classiques de traitement ou de protection de la peau choisis dans le groupe constitué par les principes actifs de protection solaire, les antioxydants, les vitamines, les agents anti-inflammatoires, les agents hydratants, les émollients, les humectants, et des mélanges de ceux-ci en une quantité efficace.

8. Composition tonifiante éclaircissante/uniformisante pour la peau selon l'une quelconque des revendications 1 à 7, dans laquelle le second agent d'éclaircissement de la peau est présent et l'efficacité d'éclaircissement de la peau de la composition est supérieure à une composition analogue contenant uniquement le second agent d'éclaircissement de la peau en une quantité équivalente à la quantité combinée d'hexylrésorcinol et du second agent d'éclaircissement de la peau.

9.  Utilisation d'une composition tonifiante éclaircissante/uniformisante pour la peau comprenant (i) une quantité efficace pour l'éclaircissement de la peau d'hexylrésorcinol hautement purifié, (ii) facultativement d'environ 0,01 à environ 20 % en poids d'au moins un autre agent d'éclaircissement de la peau, et (iii) un véhicule acceptable d'un point de vue dermatologique, dans laquelle ledit hexylrésorcinol hautement purifié a une pureté d'au moins 99 % en poids et une teneur en résorcinol, le cas échéant, non supérieure à 0,1 % % en poids, dans une méthode cosmétique d'éclaircissement de la couleur de la peau et/ou fournissant un teinte plus uniforme à la couleur de la peau.

10. Utilisation selon la revendication 9, dans laquelle la composition tonifiante éclaircissante/uniformisante pour la peau est appliquée sur les zones à éclaircir en une quantité suffisante pour fournir un revêtement léger sur la peau au moins deux fois par jour jusqu'à l'obtention de l'effet d'éclaircissement de la peau souhaité.

11. Utilisation selon la revendication 9 ou 10, dans laquelle la composition est appliquée sur les zones de la peau à protéger contre un brunissement dû à l'âge, à un régime ou à une grossesse.

12. Composition tonifiante éclaircissante/uniformisante pour la peau selon l'une quelconque des revendications 1 à 8, pour une utilisation sur les zones de la peau à protéger d'un brunissement de la peau dû à une lumière UV, une exposition au soleil et des conditions physiques et/ou physiologiques choisies parmi un traumatisme, une inflammation, une thérapie au laser, un médicament ou un traitement pharmaceutique.

**EP 2 152 685 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6649150 B, Chaudhuri
- US 6969509 B, Chaudhuri
- US 5670154 A, Hara
- US 5880314 A, Shinomiya
- US 4959393 A
- JP 5004905 A, Hamazaki
- WO 2006049184 A, Fukunishi
- JP 2001010925 A, Seto
- US 5468472 A, LaGrange
- US 6875425 B, Harichian
- US 6852310 B, Harichian
- JP 1125563 A, Sakai
- US 20060257340 A, Nair
- US 6878381 B
- US 6933319 B
- US 6852747 B
- US 6828460 B
- US 6797731 B
- US 6590105 B
- US 6541473 B
- US 6132740 A
- US 6863897 B
- US 6858217 B
- US 20060129002 A1
- US 6159485 A

- US 7186404 B, Gonzalez
- US 7175834 B, Aust
- US 7172754 B, Roseaver
- US 7175835 B
- US 7101536 B, Mongiat
- US 7078022 B, Maniscalco
- US 5175340 A, Forestier
- US 5567418 A
- US 5538716 A
- US 5951968 A
- US 5670140 A
- US 7150876 B, Chaudhuri
- US 6831191 B
- US 6602515 B
- US 7166273 B
- US 6936735 B
- US 6699463 B
- US 6165450 A, Chaudhuri
- US 6962692 B, Bonda
- US 5830441 A, Wang
- US 6124268 A, Ghosal
- US 20050048008 A1, Gupta
- US 4185100 A, Rovee
- US 4338293 A, Holick
- WO 11803188 A

**Non-patent literature cited in the description**

- **K JONES ; J HUGHES ; M HONG ; Q JIA ; S ORN-DORFF.** Modulation of melanogenesis by aloeosin: a competitive inhibitor of tyrosinase. *Pigment Cell Research,* 2002, vol. 15, 335-340
- **SH LEE ; SY CHOI ; H KIM ; JS HWANG ; BG LEE.** Mulberoside F isolated from the leaves from the leaves of Murus alba inhibits melanin biosynthesis. *Bio Pham Bull,* 2002, vol. 25, 1045-1048
- **O NERYA ; R MUSA ; S KHATIB ; S TAMIR ; J VAYA.** Chalcones as potent tyrosinase inhibitors: the effect of hydroxyl positions and numbers. *Phytochem,* 2004, vol. 65, 1389-1395
- **Y MASAMOTO ; Y MURATA ; K BABA ; Y SHIMOISHI ; M TADA ; K TAKAHATA.** Inhibitory effects of esculetin on melanin biosynthesis. *Biol Pharm Bull,* 2004, vol. 27, 422-425

- **Y YOKOTO ; H NISHIO ; Y KUBOTA ; M MI-ZOGUCHI.** The inhibitory effect of glabridin from licorice extracts on melanogenesis and inflammation. *Pigment Cell Research,* 1998, vol. 11, 355-361
- **JK NO ; DY SOUNG ; YJ KIM ; KH SHIM ; YS JUN ; SH RHEE ; R YOKOZAWA ; HY CHUNG.** Inhibition of tyrosinase by green tea components. *Pharmacol Letters,* 1999, vol. 65, 241-246
- **O NERYA ; J VAYA ; R MUSA ; S IZRAEL ; R BEN-ARIE ; S TAMIR.** Glabrene and Isoliquiritigenin as tyrosinase inhibitors fro Licorice roots. *J Agr Food Chem,* 2003, vol. 51, 1201-1207
- **I KUBO ; I KINST-HORI ; SK CHAUDHURI ; Y KUBO ; Y SANCHEZ ; T OGURA.** Flavonols from Heterotheca inuloides: Tyrosinase inhibitory activity and structural criteria. *Bioorganic & Medicinal Chemistry,* 2000, vol. 8, 1749-1755

- **NH SHIN ; SY RYU ; EJ CHOI ; SH KANG ; IM CHANG ; KR MIN ; R KIM.** Oxyresveratrol as the potent inhibitor on dopa oxidase activity on mushroom tyrosinase. *Biochem Biophys Res Commun,* 1998, vol. 243, 801-803
- **YM KIM ; J YUN ; CK LEE ; H LEE ; KR MIN ; Y KIM.** Oxyresveratrol and hydroxystilbene compounds. *J Biol Chem,* 2002, vol. 277, 16340-16344
- **RK CHAUDHURI ; Z LASCU ; G PUCCETTI.** Inhibitory effects of Phyllanthus emblica tannins on melanin synthesis. *Cosmetics & Toiletries,* 2007, vol. 122 (2), 73-80
- **LAW et al.** *Br. J. Pharmac.,* 1977, vol. 59 (4), 591-597
- **KAIDBEY.** *J. Invest. Dermatoloy,* 1976, vol. 66, 153-156
- **GRUBER et al.** *Clinical Pharm. and Therapeut.,* 1971, vol. 13 (1), 109-113
- Appraisal of the Safety of Chemicals in Food, Drugs and Cosmetics. The Association of Food and Drug Officials of The United States